## Europäisches Patentamt

## European Patent Office

## Office européen des brevets

(19)

(11) **EP 0 494 177 B1**

(12)

# EUROPÄISCHE PATENTSCHRIFT

(45) Veröffentlichungstag und Bekanntmachung des Hinweises auf die Patenterteilung:
**06.03.1996 Patentblatt 1996/10**

(21) Anmeldenummer: **90913700.2**

(22) Anmeldetag: **21.09.1990**

(51) Int Cl.$^6$: **C11C 3/04**

(86) Internationale Anmeldenummer:
**PCT/EP90/01611**

(87) Internationale Veröffentlichungsnummer:
**WO 91/05034 (18.04.1991 Gazette 1991/09)**

(54) **KONTINUIERLICHES VERFAHREN ZUM HERSTELLEN NIEDERER ALKYLESTER**

CONTINUOUS PROCESS FOR PRODUCING LOWER ALKYL ESTERS

PROCEDE DE FABRICATION CONTINUE D'ESTERS INFERIEURS D'ALKYL

(84) Benannte Vertragsstaaten:
**AT BE CH DE DK ES FR GB IT LI LU NL SE**

(30) Priorität: **29.09.1989 DE 3932514**

(43) Veröffentlichungstag der Anmeldung:
**15.07.1992 Patentblatt 1992/29**

(73) Patentinhaber: **Henkel Kommanditgesellschaft auf Aktien**
**D-40191 Düsseldorf (DE)**

(72) Erfinder:
* **ASSMANN, Georg**
**D-4053 Jüchen (DE)**
* **BLASEY, Gerhard**
**D-4000 Düsseldorf (DE)**
* **GUTSCHE, Bernhard**
**D-4010 Hilden (DE)**
* **JEROMIN, Lutz**
**D-4010 Hilden (DE)**
* **RIGAL, Jean**
**F-31360 Saint-Martory (FR)**
* **ARMENGAUD, René**
**F-31360 Saint-Martory (FR)**
* **CORMARY, Bernard**
**F-31260 Salies du Salat (FR)**

(56) Entgegenhaltungen:
EP-A- 0 131 991          DE-A- 3 020 612

**Beschreibung**

Die Erfindung betrifft ein kontinuierliches Verfahren zum Herstellen niederer Alkylester, insbesondere Methylester, bei Temperaturen bis zu 100 °C und Drücken bis zu 10 bar, durch ein mindestens zweistufiges Umsetzen von Fettsäuretriglyceriden, einen Anteil an freier Fettsäure (ffa) von kleiner 1 % beinhaltend, mit einem niederen Alkohol, insbesondere Methanol, in Gegenwart eines homogenen alkalischen Katalysators, wobei nach jeder Stufe das entstandene Glycerin aus dem Reaktionsgemisch abgetrennt wird.

Die Herstellung von Alkylestern und Glycerin aus natürlichen Fetten und Ölen ist ein schon seit Jahrzehnten durchgeführter chemischer Prozeß. Die beiden hauptsächlich angewandten Verfahren sind die Hochdruckumesterung bei Drücken bis zu 90 bar und Temperaturen bis 250 °C und die Niederdruckumesterung bei 1 bis 10 bar und Temperaturen unter 100 °C.

Bei der Niederdruckumesterung werden entsäuerte Öle und Fette mit einem niedrigen Anteil an freien Fettsäuren eingesetzt. Beim Einsatz eines alkalischen Katalysators wird der Katalysator durch die freie Fettsäure neutralisiert, so daß dessen Aktivität damit nicht mehr zur Verfügung steht. Als alkalische Katalysatoren werden Natriummethylat, Natriumhydroxid, Kaliumhydroxid und andere eingesetzt. Weitere Katalysatoren finden sich im Artikel in J. scient. ind. Res. Vol. 33, April 1974, S. 178 - 187.

Bei der einfachsten Ausgestaltung des Verfahrens, der Niederdruckumesterung, reagiert das angesetzte Gemisch wenige Minuten bis mehrere Stunden lang in einem Rührkessel. Anschließend erfolgt die Dekantation der leichten Phase, des Alkylesters, und der Glycerinphase im gleichen Kessel. Eine kurze Beschreibung befindet sich in der GB-PS 634411 und in der US-PS 2360844.

Nachteilig an dieser einfachen Vorgehensweise ist ein hoher Bedarf an Alkohol und alkalischem Katalysator in der Umesterung, um einen hohen Umsatz zu erreichen, da die Umesterung eine Gleichgewichtsreaktion ist. Der hohe Überschuß an Katalysator und Alkohol erschwert einerseits die Dekantation von Glycerin mit Alkohol und andererseits die eigentliche angeschlossene Glyceringewinnung. In der Glyceringewinnung müssen nämlich die aus dem Katalysator entstandenen Seifen durch anorganische Säure unter Wasserzusatz gespalten werden. Die Fettsäuren können dann durch Dekantation vom Rohglycerin getrennt werden.

Um bei einem geringeren Einsatz an Alkohol und alkalischem Katalysator dennoch eine ausreichend hohe Ausbeute zu erzielen, ist die Umesterung zweistufig durchzuführen. So wird nach der DE-PS 3020612 bei einer diskontinuierlichen, im Rührkessel vorgenommenen Niederdruck-Umesterung nach einer ersten Reaktionsstufe und einer anschließenden Dekantation das als Unterphase vorliegende Glycerin aus dem Reaktionsgemisch entfernt und die Oberphase nochmals umgeestert. Zur Glycerinentfernung wird nach dem 2. Reaktionsschritt Wasser zur besseren Abscheidung zugegeben, was zwar zu einer guten Glycerinentfernung führt, bezüglich der Glycerinaufarbeitung einen zusätzlichen Aufwand bedeutet.

Ein demgegenüber verbessertes Niederdruck-Umesterungsverfahren findet sich im Zeitschriftenartikel Seifen, Öle, Fette, Wachse, 1988, Seiten 595 bis 600, insbesondere Figur 3 auf Seite 596 mit zugehöriger Beschreibung. Es handelt sich um ein kontinuierliches und zweistufiges Verfahren. Die Einsatzstoffe Öl mit niedrigem ffa, Methanol und alkalischer Katalysator werden von einer Pumpe in eine erste Reaktionsschlaufe eindosiert. Von einer Zentrifugalpumpe wird das Reaktionsgemisch in einen ersten Reaktor gefördert. Die Zentrifugalpumpe dient dabei gleichzeitig zum innigen Durchmischen des Reaktionsgemisches. Nach der entsprechenden Verweilzeit im Reaktor fließt das Gemisch in einen statischen stehenden Abscheider. Nach der Dekantation wird die Oberphase zurück zur Zentrifugalpumpe geführt und die Unterphase, die fast das gesamte entstandene Glycerin zusammen mit restlichem Methylester und nicht umgesetzten Triglyceriden enthält, wird zu einer Zentrifuge zum besseren Abscheiden des Glycerins geleitet. Die vom Glycerin befreite Oberphase durchläuft dann eine zweite Reaktionsschlaufe. Jede Reaktionsschlaufe besteht aus je einer Zentrifugalpumpe, einem Reaktor und einem statischen Abscheider. Die Pumpe in jeder Reaktionsschlaufe ist notwendig, da das reagierte Gemisch in jeder Schlaufe rückgeführt wird. Da die Trennung in diesem bekannten Verfahren innerhalb des statischen Abscheiders nur unvollkommen ist, ist zum Abtrennen von Glycerin die genannte Zentrifuge notwendig.

Der Erfindung liegt die Aufgabe zugrunde, ein kontinuierliches Verfahren zum Herstellen niederer Alkylester mit hoher Ausbeute der eingangs genannten Art zu schaffen, das weniger Instandhaltungs-, Investitions- und Energiekosten erfordert und einfach und sicher zu betreiben ist.

Diese Aufgabe wird erfindungsgemäß dadurch gelöst, daß die Reaktion mindestens zweistufig in einem Rohrreaktor durchgeführt wird. Das Reaktionsgemisch durchläuft nur einmal den Rohrreaktor und nachgeschalteten liegenden statischen Abscheider in jeder Stufe. In den Rohrreaktoren muß eine gute Vermischung durch Turbulenz vorliegen, hierfür wird als Kennzahl die Reynoldszahl

$$Re = \frac{\rho v \, d}{\eta}$$

mit $\rho$ als Gemischdichte, $v$ als durchschnittliche Rohrgeschwindigkeit, $d$ als innerer Rohrdurchmesser und $\eta$ als Gemischviskosität herangezogen. Die Re-Zahl wird für den Eingang des 1. Rohrreaktors berechnet.

Die Turbulenz muß in jedem Fall gegeben sein. Ab einer Reynolds-zahl von 2300 setzt der turbulente Bereich ein. Für viele Anwendungsfälle ist eine Reynoldszahl von 4000 dann ausreichend. Ein sehr sicherer Betrieb wurde bei einer Reynoldszahl von über 10;000 gefunden.

Bei der Berechnung der Reynoldszahl wird die Dichte nach der bekannten Beziehung bei Reaktionstemperatur berechnet. Dabei wird die Beziehung für die mittlere Dichte eines pseudo-einphasigen Gemisches verwendet:

$$\frac{1}{\rho} = \sum_i \frac{w_i}{\rho_i}$$

wobei $\rho_i$ die Dichte von Komponente i in kg/m$^3$ und $w_i$ der Massenanteil dieser Komponente ist.

Alternativ kann die Dichte auch mit einem Dichtemeßgerät bestimmt werden. Für die Viskosität gilt eine entsprechende Beziehung. Neben der experimentellen Bestimmung von $\eta$ mit einem Viskosimeter kann die Pseudo-Gemisch-Viskosität nach folgender Gleichung berechnet werden:

$$\ln \eta = \sum_i x_i \ln \eta_i$$

wobei $\eta_i$ die Viskosität der Komponente i in kg/ms und $x_i$ der Molenbruch der Komponente i ist.

Die Rohrlänge erhält man aus reaktionskinetischen Untersuchungen, aus denen sich die Verweilzeit für einen geforderten Umsatz ergibt. Da die Geschwindigkeit im Rohr festgelegt ist, wird die Rohrlänge aus der benötigten Verzweilzeit errechnet. Die Verweilzeit wird im Rohr auf 1 - 10 Minuten eingestellt, wobei bei der Methylesterproduktion ca. 2 - 5 Minuten vorteilhaft sind. Der begrenzende Faktor für die Länge des Rohrreaktors kann der Druckverlust im Rohr sein. Für den Fall der Herstellung von Alkylestern aus Ölen und Fetten mit Natriummethylat als Katalysator ist nur ein geringer Druckverlust von kleiner 1 bar bei Drücken von 2 bis 10 bar festgestellt worden.

Neben einer guten Vermischung der Einsatzstoffe ist für die Umesterungsreaktion besonders wichtig, daß kein Glycerin (Reaktionsprodukt) mit Einsatzöl, Methanol und neuem Katalysator in Kontakt kommt (Rückvermischung), da hierdurch das Glycerin mit dem Ester teilweise zum Mono-, Di- und Triglycerid zurückreagiert. Diese Glyceride müssen erneut abgebaut werden, was einen erhöhten Aufwand mit relativer Umsatzverschlechterung bedeutet. Es wird also eine Verweilzeitverteilung gefordert, die der im Rohrreaktor entspricht. Aus Laborversuchen mit kontinuierlich betriebenem Rührkessel ist bekannt, daß die Umesterungsreaktion bei gleichem Alkoholüberschuß und gleicher Katalysatorkonzentration für Kokosöl in ca. 15 Minuten den Umsatz erreicht, der in einem entsprechendem Rohrreaktor nach 2 Minuten Verweilzeit erzielt wird.

Vorteilhaft ist, wenn das Trennen der beiden Flüssigkeitsphasen mit rohrförmigen, liegenden Abscheidern durchgeführt wird. Das Reaktionsgemisch strömt an dem einen Ende des Abscheiders in diesen ein, und am entgegengesetzten Ende fließen Oberphase und Unterphase (Glycerin) getrennt ab. In diesem Fall findet zu Beginn des Einströmens des Reaktionsgemisches die Nachreaktion bei gleichzeitiger Abscheidung statt. Gegen Ende des Abscheiders sind Nachreaktion und Abscheidung abgeschlossen. Da es sich um einen rohrförmigen Abscheider handelt, tritt keine Rückvermischung von abgeschiedenem Ester mit noch nachzureagierendem Ester auf. Dieser langsame Übergang von Nachreaktion und Abscheidung wäre in rührkesselartigen stehenden Abscheidern im kontinuierlichen Betrieb nicht ohne Austrag von Glycerinphase im Ester möglich. Durch die Nachreaktion wird die Ausbeute an Methylester um bis zu 20 % gesteigert.

Es wird vorgeschlagen, daß die Verweilzeit des Reaktionsgemisches in den Abscheidern 0,1 bis 5 Stunden beträgt und daß das Reaktionsgemisch in den Abscheidern auf Reaktionstemperatur gehalten wird.

Im Falle der Methylesterherstellung ist eine Abscheidezeit von 15 Minuten bis 2 Stunden einzustellen.

Der gewonnene Alkylester enthält noch Reste an Alkohol und Katalysator. In der zweiten Stufe, die nach den gleichen Gesichtspunkten wie die erste Stufe ausgelegt wird, wird zusätzlich Alkohol und Katalysator zudosiert. Nach erfolgter Vermischung und Dekantation werden Triglycerid-Umsätze von insgesamt 98 % erreicht.

Eine weitere Einsparung an Alkohol und Katalysator wird erreicht, indem man ein dreistufiges Verfahren konzipiert. Der zusätzliche apparative Aufwand ist insbesondere bei Umesterungen mit höheren Alkoholen (Ethanol, Butanol usw.), bei denen schon bei niedrigen Umsätzen eine Glycerinphase abgetrennt werden kann, vorteilhaft und bringt weitere Vorteile bei der Herstellung der entsprechenden Alkylester. Dabei ist eine weitere Reduzierung des Alkoholüberschusses bzw. des Katalysatoreinsatzes möglich.

Der Alkoholeinsatz kann bei Aufrechterhaltung eines hohen Umsatzes im Vergleich zu bisherigen Verfahren verringert werden, wenn in beiden Stufen von einem Alkohol-Triglycerid-Verhältnis von 4,5 bis 7,5 mol/mol Triglycerid, insbesondere bei der Methylesterherstellung von 5,1 bis 6,9 mol/mol Triglycerid, ausgegangen wird.

Bei Aufrechterhaltung des hohen Umsatzes wird in diesem neuen Verfahren der Katalysator- und Alkoholeinsatz gering gehalten: Die Gesamtkonzentration des Katalysators (z. B. Natriummethylat) in beiden Stufen zusammen ist geringer als 0,25 Ma % - bezogen auf neutrales Triglycerid - und die insgesamt einzusetzende Menge an Alkohol und Katalysator wird in Abhängigkeit von Qualität und Kettenlängenverteilung des umzuesternden Triglycerids derart auf beide Stufen aufgeteilt, daß in jeder Stufe ein Umsatz von mindestens 85 % erzielt wird. Hierbei kann der Katalysatoreinsatz in der 2. Stufe bei $\leq$ 0,05 % gehalten werden.

Zum Vorvermischen des Reaktionsgemisches wird vorgeschlagen, daß das Reaktionsgemisch durch statische Mischer geführt wird.

Beispiele

1. Umesterung von Rüböl

In einer Technikumsanlage wurde entsäuertes Rüböl mit ca. 150 1/h in einen Rohrreaktor bei 75 °C und 3 bar eingespeist. Methanol und Natriummethylat wurden als Lösung bei 20 °C zugegeben. Das Verhältnis von Methanol zu Öl betrug für die erste Stufe 0,2 und für die zweite Stufe 0,04. Der Katalysator wurde, bezogen auf Neutralöl, mit 0,2 % bzw. 0,04 % (Massenprozent) dosiert. Bei einer Verweilzeit von 2 Minuten erfolgte ein Umsatz von 90 %. Nach der Abscheidung von Glycerin in 2 Stunden wurde nach der zweiten Stufe ein Gesamtumsatz von 98 % erzielt. Die eingestellte Re-Zahl betrug 2700 (Eintritt 1. Reaktor).

2. Umesterung von Kokosöl

In einer zweistufigen Umesterungsanlage mit Rohrreaktoren und Zwischenabscheidung wurden 5000 l/h Kokosöl mit geschupptem Natriumhydroxid und Methanol umgeestert, wobei der Katalysator in Methanol gelöst worden war. Das Verhältnis von Methanol zu Öl betrug in der ersten Stufe 0,3 und in der zweiten Stufe 0,04. Das Verhältnis von Katalysator zu Neutralöl wurde in der ersten Stufe zu 0,24 % und in der zweiten Stufe zu 0,04 % eingestellt. Nach der ersten Stufe wurde ein Umsatz von 88 % festgestellt, so daß der Gesamtumsatz nach der zweiten Stufe 98 % betrug. Die Re-Zahl am Eintritt des 1. Rohrreaktors betrug 7 500.

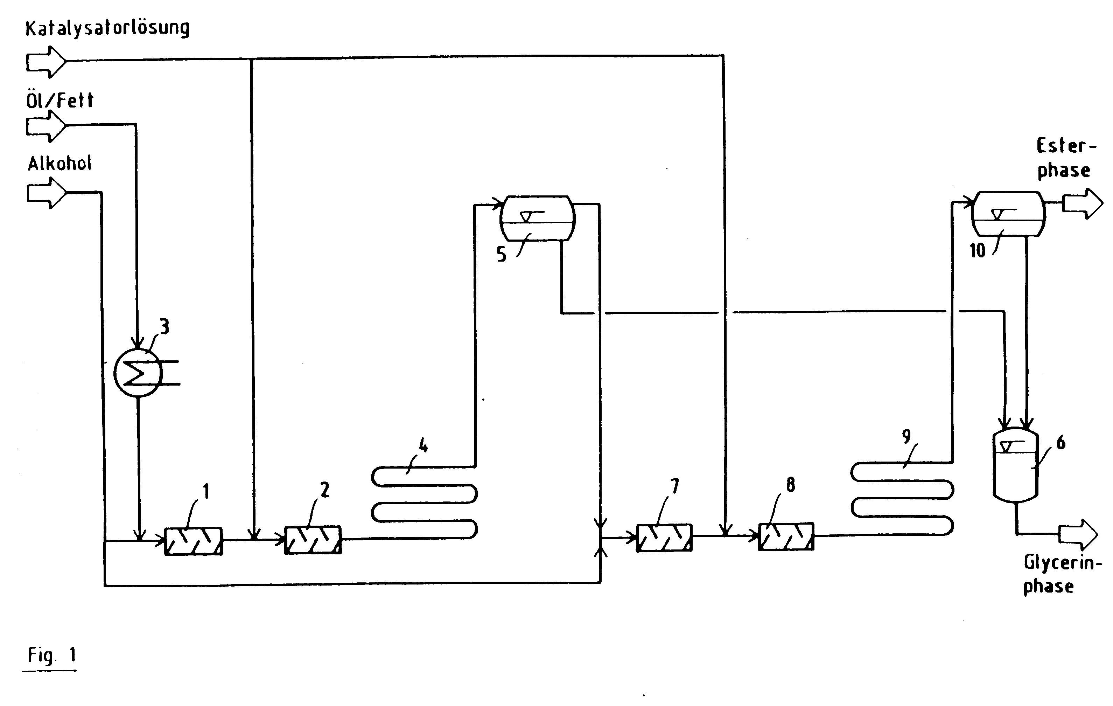

Im folgenden werden die Ausführungsbeispiele der Erfindung anhand der einzigen Figur 1 näher erläutert.

Figur 1 zeigt den Aufbau einer Anlage zum Betreiben des erfindungsgemäßen Verfahrens. Aus Vorlagen werden Öl, Methanol und Katalysatorlösung durch zwei statische Mischer (1 und 2) in den ersten Reaktionsabschnitt gefördert. Das natürliche Öl, also Triglycerid, wird im Wärmetauscher (3) erhitzt und mit kaltem Methanol im ersten statischen Mischer (1) intensiv vermengt. Danach wird der Katalysator zugemischt, so daß die Reaktion im Rohrreaktor (4) ablaufen kann. Das Reaktionsgemisch gelangt in den ersten liegenden statischen Abscheider (5), wo es nachreagiert und gegen Ende der Verweilzeit im Abscheider in zwei Phasen aufgetrennt ist. Die Unterphase wird zu einer Glycerinvorlage (6) geleitet; die Oberphase durchläuft eine weitere Stufe mit statischem Mischer (7 und 8), einem zweiten Rohrreaktor (9) und einem zweiten liegenden Abscheider (10). Die Einspeisung von Methanol und Katalysatorlösung erfolgt für die zweite Stufe (7 und 10) wie in der ersten Stufe. Die Unterphase des zweiten statischen Abscheiders wird ebenfalls in die Glycerinvorlage (6) geführt, worauf sich die weitere Reinigung der Glycerinphase anschließt. Die Oberphase wird als Methylesterphase gewonnen.

**Bezugszeichenliste**

| 1, 2, 8, 9 | statischer Mischer |
| 3 | Wärmetauscher |
| 4, 9 | Rohrreaktor |
| 5, 10 | stat. Abscheider |
| 6 | Glycerinvorlage |

**Patentansprüche**

1. Kontinuierliches Verfahren zum Herstellen niederer Alkylester, insbesondere Methylester, bei Temperaturen bis zu 100 °C und Drücken bis zu 10 bar, durch ein mindestens zweistufiges Umsetzen von Fettsäuretriglyceriden mit einem Gehalt an freien Fettsäuren kleiner als 1 % mit einem niederen Alkohol, insbesondere Methanol, in Gegenwart eines homogenen alkalischen Katalysators, wobei nach jeder Stufe das entstandene Glycerin abgetrennt wird,

   **dadurch gekennzeichnet**,
   daß das Reaktionsgemisch in jeder Stufe nur einen Rohrreaktor (4, 9) und einen nachgeschalteten statischen Abscheider (5, 10) durchläuft, daß als Reaktoren Rohrreaktoren (4, 9) eingesetzt werden und daß die Reynolds-Zahl der Strömung des Reaktionsgemisches am Eingang des ersten Rohrreaktors (4) größer als 2300, insbesondere größer als 4000 ist und ganz besonders größer als 10 000 ist, wobei

   $$Re = \frac{\rho v\, d}{\eta}$$

   die Reynoldszahl und $\rho$ die Gemischdichte, v die lineare Rohrgeschwindigkeit, d der innere Rohrdurchmesser und $\eta$ die Gemischviskosität ist.

2. Verfahren nach Anspruch 1,

   **dadurch gekennzeichnet**,
   daß die Verweilzeit des Reaktionsgemisches in den Abscheidern (5, 10) 0,1 bis 5 Stunden beträgt, und daß das Reaktionsgemisch in den Abscheidern (5, 10) auf Reaktionstemperatur gehalten wird.

3. Verfahren nach Anspruch 2,

   **dadurch gekennzeichnet**,
   daß das Verfahren mit rohrförmigen, liegenden Abscheidern (5, 10) durchgeführt wird und das Reaktionsgemisch an dem einen Ende des rohrförmigen Abscheiders (5, 10) in diesen einströmt, am entgegengesetzten Ende in Oberphase (Ester) und Unterphase (Glycerin) getrennt ist und separat abfließt.

4. Verfahren nach einem der Ansprüche 1 bis 3,

**dadurch gekennzeichnet**,
daß die Verweilzeit in den Rohrreaktoren jeweils 1 bis 10 Minuten, insbesondere 2 bis 5 Minuten bei der Herstellung von Methylester, beträgt.

5. Verfahren nach einem der Ansprüche 1 bis 4,

**dadurch gekennzeichnet**,
daß in beiden Stufen Alkohol und Triglycerid in einem Verhältnis von 4,5 bis 7,5 mol/mol Triglycerid, insbesondere bei der Methylesterherstellung von 5,1 bis 6,9 mol/mol Triglycerid, eingesetzt werden

6. Verfahren nach einem der Ansprüche 1 bis 5,

**dadurch gekennzeichnet**,
daß die Gesamtkonzentration des Katalysators in beiden Stufen zusammen geringer als 0,25 Ma %, bezogen auf neutrales Triglycerid, beträgt und daß die insgesamt einzusetzende Menge an Alkohol und Katalysator in Abhängigkeit von Qualität und Kettenlängenverteilung des umzuesternden Triglycerids derart auf beide Stufen aufgeteilt wird, daß in jeder Stufe ein Umsatz von mindestens 85 % erzielt wird.

7. Verfahren nach einem der Ansprüche 1 bis 6,

**dadurch gekennzeichnet**,
daß zum Vorvermischen das Reaktionsgemisch durch mindestens einen statischen Mischer (1, 2, 7, 8) geführt wird.

## Claims

1. A continuous process for the production of lower alkyl esters, more particularly methyl esters, at temperatures of up to 100°C and under pressures of up to 10 bar by reaction of fatty acid triglycerides containing less 1% of free fatty acids with a lower alcohol, more particularly methanol, in at least two stages in the presence of a homogeneous alkaline catalyst, the glycerol formed being removed from the reaction mixture after each stage, characterized in that the reaction mixture passes through only one tube reactor (4,9) and a following static separator (5,10) in each stage, in that tube reactors (4,9) are used as the reactors and in that the Reynolds number of the flow of the reaction mixture at the entrance of the first tube reactor (4) is greater than 2,300, preferably greater than 4,000 and more preferably greater than 10,000,

$$Re = \frac{\rho v \, d}{\eta}$$

being the Reynolds number where $\rho$ is the density of the mixture, $v$ is the linear tube velocity, $d$ is the tube diameter and $\eta$ is the viscosity of the mixture.

2. A process as claimed in claim 1, characterized in that the residence time of the reaction mixture in the separators (5,10) is 0.1 to 5 hours and in that the reaction mixture is kept at the reaction temperature in the separators (5,10).

3. A process as claimed in claim 2, characterized in that the process is carried out with tubular horizontal separators (5,10) and in that the reaction mixture flows into the tubular separator (5,10) at one end and is separated into an upper phase (ester) and lower phase (glycerol) at the opposite end, the two phases flowing off separately.

4. A process as claimed in any of claims 1 to 3, characterized in that the residence time in the tube reactors is 1 to 10 minutes and more particularly 2 to 5 minutes in the production of methyl ester.

5. A process as claimed in any of claims 1 to 4, characterized in that, in both stages, alcohol and triglyceride are used in a ratio of 4.5 to 7.5 moles/mole triglyceride and, particularly in the production of methyl ester, in a ratio of 5.1 to 6.9 moles/mole triglyceride.

6. A process as claimed in any of claims 1 to 5, characterized in that the total concentration of catalyst in both stages taken together is less than 0.25% by weight, based on neutral triglyceride, and in that the total quantity of alcohol and catalyst is divided between both stages, depending on the quality and chain length distribution of the triglyceride to be transesterified, in such a way that a conversion of at least 85% is obtained in each stage.

7. A process as claimed in any of claims 1 to 5, characterized in that, for preliminary mixing, the reaction mixture is passed through at least one static mixer (1,2,7,8).

## Revendications

1. Procédé en continu d'obtention d'esters d'alcoyle inférieur, en particulier de l'ester méthylique, à des températures allant jusqu'à 100°C et à des pressions allant jusqu'à 10 bars, à l'aide d'une réaction au moins en deux étapes de triglycérides d'acide gras ayant une teneur en acide gras libre inférieure à 1 %, avec un alcool inférieur, en particulier le méthanol, en présence d'un catalyseur alcalin homogène tandis que, après chaque étape, le glycérol qui s'est formé est séparé, caractérisé en ce que le mélange réactionnel à chaque étape parcourt seulement un réacteur tubulaire (4, 9) et un séparateur statique post-connecté (5, 10) en ce que comme réacteurs on met en jeu des réacteurs tubulaires (4, 9) et en ce que le nombre de Reynolds du courant de mélange réactionnel à l'entrée du premier réacteur tubulaire (4) est plus grand que 2300, en particulier plus grand que 4000 et est d'une manière tout à fait particulière plus grand que 10.000, tandis que :

$$Re = \frac{\rho v\, d}{\eta}$$

est le nombre de Reynolds et
avec

$\rho$    comme densité du mélange
$v$    comme vitesse moyenne dans le tube
$d$    comme diamètre interne du tube et,
$\eta$    comme viscosité du mélange.

2. Procédé selon la revendication 1, caractérisé en ce que le temps de séjour du mélange réactionnel dans les séparateurs (5, 10) s'élève de 0,1 à 5 heures, et en ce que le mélange réactionnel est maintenu dans les séparateurs (5, 10) à la température de réaction.

3. Procédé selon la revendication 2, caractérisé en ce que le procédé est exécuté avec des séparateurs (5, 10) de forme tubulaire, disposés horizontalement, et en ce que le mélange réactionnel à une des extrémités du séparateur de forme tubulaire (5,10) s'écoule dans celui-ci, est séparé à l'extrémité opposée en une phase supérieure (ester) et en une phase inférieure (glycérol), que l'on fait se déverser séparément.

4. Procédé selon l'une des revendications 1 à 3, caractérisé en ce que le temps de séjour dans les réacteurs tubulaires s'élève respectivement de 1 à 10 minutes, en particulier de 2 à 5 minutes au cours de la préparation de l'ester méthylique.

5. Procédé selon l'une des revendications 1 à 4, caractérisé en ce que dans les deux étapes l'alcool et le triglycéride sont mis en oeuvre dans un rapport allant de 4,5 à 7,5 mol/mol de triglycéride, en particulier pour la préparation de l'ester méthylique de 5,1 à 6,9 mol/mol de triglycéride.

6. Procédé selon l'une des revendications 1 à 5, caractérisé en ce que la concentration globale du catalyseur dans les deux étapes s'élève ensemble à moins de 0,25 % en masse, rapporté au triglycéride neutre, et en ce que la quantité à mettre en jeu au total en alcool et en catalyseur est répartie en fonction de la qualité et de la distribution des longueurs de chaîne du triglycéride à transestérifier dans les deux étapes, de façon qu'à chaque étape un taux de conversion d'au moins 85 % soit atteint.

7. Procédé selon l'une des revendications 1 à 6, caractérisé en ce que pour le prémélange le mélange réactionnel est conduit à travers au moins un mélangeur statique (1, 2, 7, 8).

Katalysatorlösung

Öl/Fett

Alkohol

Esterphase

EP 0 494 177 B1

7

3

5

10

4

1 2

9 6

7 8

Glycerinphase

Fig. 1